# EUROPEAN PATENT SPECIFICATION

(11) **EP 2 736 507 B1**
(45) Date of publication and mention of the grant of the patent: **07.11.2018**
(21) Application number: 12759243.4
(22) Date of filing: 27.07.2012
(51) Int. Cl.: A61K 31/385, A61K 9/08, A61K 47/02, A61K 47/10, A61P 3/10, A61P 9/10, A61P 35/00, A61P 25/00, A61P 25/28, A61P 9/00, A61K 9/00, A61K 9/10, A61K 31/205, A61K 31/198, A61K 31/122, A61K 9/48

(54) **BASIC ALPHA LIPOIC ACID SOLUTION AND ITS USES**
BASISCHE ALPHA-LIPONSÄURELÖSUNG UND VERWENDUNGEN DAVON
SOLUTION D'ACIDE ALPA-LIPOÏQUE BASIQUE ET UTILISATION ASSOCIÉE

(30) Priority: 29.07.2011 IT MI20111452
(43) Date of publication of application: 04.06.2014
(73) Proprietor: ITAPHARMA S.R.L., 56121 Pisa (IT)
(72) Inventor: BRUFANI, Mario, 00040 Castelgandolfo (IT); FIGLIOLA, Rocco, 82025 Montefalcone di Valfortore (IT); LAGRASTA, Bianca Maria, 00191 Roma (IT); MARZELLA, Rolando, 00144 Roma (IT); MEDICI, Ilaria, 00149 Roma (IT); SILVESTRI, Silvio, 00171 Roma (IT)
(74) Representative: Cattaneo, Elisabetta
(86) International application number: PCT/IB2012/053840
(87) International publication number: WO 2013/018008

(56) References cited:
- EP-A1- 0 318 891
- EP-A1- 2 241 314
- EP-A2- 0 427 247
- YALKOWSKY S H ET AL: "SOLUBILIZATION BY COSOLVENTS I: ORGANIC SOLUTES IN PROPYLENE GLYCOL-WATER MIXTURES", JOURNAL OF PHARMACEUTICAL SCIENCES, AMERICAN PHARMACEUTICAL ASSOCIATION, WASHINGTON, US, vol. 74, no. 4, 1 April 1985 (1985-04-01), pages 416-421, XP008058020, ISSN: 0022-3549, DOI: 10.1002/JPS.2600740410

## Description

### FIELD OF THE INVENTION

The present invention concerns a solution having a pH 7.0 to 9.5, comprising alpha lipoic acid or its enantiomer, an inorganic compound selected from hydroxide, carbonate, bicarbonate and mixtures thereof, of sodium, potassium, calcium, magnesium, ammonium and mixtures thereof, and a solvent, said solvent being a mixture of water and a polyol selected from propylene glycol, glycerol, polyethylene glycol, polypropylene glycol, and mixtures thereof. Said solution presents high stability of the alpha lipoic acid over time, as well as high edibility and bioavailability.

### BACKGROUND OF THE INVENTION

The alpha lipoic acid (a-LA or 1,2-dithiolane-3-pentanoic acid or 1,2-dithiolane-3-valeric acid or thioctic acid) is a cofactor of various oxidative decarboxylation reactions of alpha-keto acids, such as pyruvic acid, alpha-ketoglutaric acid, branched keto acids, and of glycine.

In its enantiomeric form *R* it is bound to multi-enzyme complexes of the oxidative decarboxylases of the alpha-keto acids (alpha-keto acid dehydrogenase), where it acts redox functions by enzymatically reducing into alpha dihydrolipoylic acid (α-DHLA) (formula B): 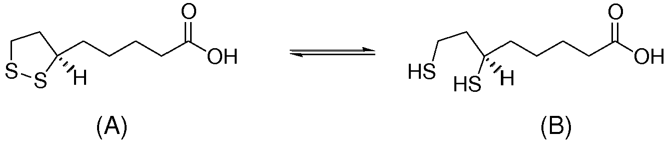 and reoxidising, again enzymatically, into α-LA (formula A).

The alpha lipoic acid also acts as carrier of acetyl residues; it in fact transfers the acetyl group that is formed by oxidative decarboxylation of the pyruvic acid on Coenzyme A. The reaction, which requires α-LA as cofactor, can be illustrated as follows:

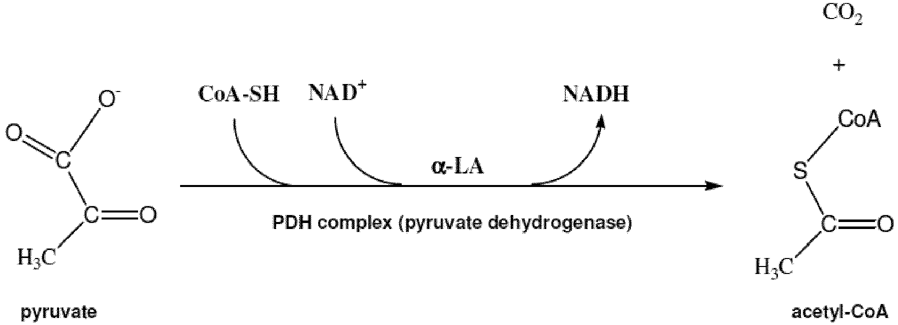

From this reaction scheme it can be seen that the oxidising agent is the NAD⁺ and that the reaction produces an equivalent of NADH. The reaction takes place in the mitochondria and is essential for initiating Krebs cycle reactions.

The α-LA, previously in racemic form and, in more recent preparations, in enantiomeric form *R,* is widely used as a food supplement, and in some Countries, as a medicine for the treatment of diabetic polyneuropathy. The basis of the pharmacological action of α-lipoic acid are not yet entirely clear, although various hypotheses have been proposed in this regard. In particular, it has been hypothesised that in neuropathic processes, α-lipoic acid has a protective action due to its redox properties that are capable of neutralising, at least in part, the damage caused by the free radicals that are generated in the peripheral nervous system of the diabetic patient (Shay et al., "Alpha lipoic acid as a dietary supplement: Molecular mechanisms and therapeutic potential" Biochimica et Biophysica acta 2009; 1149-1160; Packer L et al., "Molecular aspects of lipoic acid in the prevention of diabetes complications", Nutrition, 2001; 17:888-895).

The antioxidant action of α-lipoic acid is also in part due to its ability to restore the endogenous levels of glutathione, the values of which are drastically reduced in neuropathic patients as a result of the reduction processes of excess glucose to sorbitol and of the latter's successive reoxidation to fructose. A large number of free radicals are generated in both these reactions and it has recently been demonstrated that in the diabetic patient, these free radicals generated by the redox reactions involving glucose, lead to destruction of the nitric oxide (NO), a known vasodilator, therefore responsible for a correct supply to the peripheral nerves. A dramatic decrease of NO results into a vasoconstriction and, consequently, into an insufficient supply to the nerve tissue. It is therefore possible that this is the main cause of the damage that the excess glucose causes in the peripheral nerves of the diabetic patient. Furthermore, even the non-enzymatic glycation of a number of proteins, caused by the excess glucose, by determining a loss of protein functions, contributes to the neuropathic damage (Packer L et al., "Molecular aspects of lipoic acid in the prevention of diabetes complications", Nutrition, 2001; 17:888-895).

The literature (Amenta et al.; "Pharmacokinetics of different formulations of tioctic (alpha lipoic) acid in healthy volunteers", Clinical and Experimental Hypertension, 2008 30:767-775) confirms that pharmacokinetic studies have been carried out on humans, with the administration of lipoic acid by oral route at the doses normally used in food supplements and in pharmacological preparations (600 mg). Extremely variable values have been reported, particularly depending on the type of formulation used, however an extremely reduced bioavailability (less than 30%) was always observed. Some solid, delayed release forms were also prepared, however none of them significantly increased bioavailability and the time the lipoic acid lasts in the plasma.

The easiness with which alpha lipoic acid is metabolised by oxidation (particularly beta-oxidation) has been believed so far to be the main cause of its unfavourable pharmacokinetic characteristics. The *R*-enantiomer of α-LA, which is less toxic and is pharmacologically more active than the corresponding raceme, however presents the same unfavourable, pharmacokinetic characteristics as racemic α-LA. EP0318891 and EP0427247 disclose an injectable solution of alpha lipoic acid with no problem of bioavailability, being the administration parental.

EP2241314 describes nanoparticles of lipoic acid, said nanoparticles comprising alpha-lipoic acid.

It is therefore the object of this invention to find a way to stabilise the alpha lipoic acid in solution so as to optimise the bioavailability in oral administration and increase its preservation over time.

### SUMMARY OF THE INVENTION

The above object has been achieved by a solution for use as a medicament in oral administration as reported in claim 1, i.e. having a pH 7.0 to 9.5, and comprising alpha-lipoic acid or its enantiomer, an inorganic compound selected from hydroxide, carbonate, bicarbonate and mixtures thereof, of sodium, potassium, calcium, magnesium, ammonium and mixtures thereof, and a solvent, said solvent being a mixture of water and a polyol selected from propylene glycol, glycerol, polyethylene glycol, polypropylene glycol, and mixtures thereof.

For the purposes of the present invention, "solution" means a homogenous mixture of the different components.

The present disclosure relates to a pharmaceutical composition comprising said solution.

In a further aspect, the present invention relates to a kit comprising:
i) at least one container comprising the solution for use of the invention;
ii) at least one container comprising pharmaceutically acceptable excipients, acetyl-L-carnitine, selenium, coenzyme Q, vitamins or a mixture thereof; and
iii) an illustrative leaflet,
for the separate, sequential or simultaneous oral administration.

In a yet further aspect, the present invention concerns the use of the said solution in the treatment of diabetes, neuropathies, diabetic nephropathy, diabetic retinopathy, cataracts, obesity, stroke, traumas, reperfusion injuries, cardiomyopathy, mitochondrial decay, canicolar syndromes, radiculopathies, aging, vascular malfunctions in the elderly, cancer, osteoporosis, Alzheimer's disease, Parkinson's disease, hepatic fibrosis or muscular fatigue.

### BRIEF DESCRIPTION OF THE DRAWING

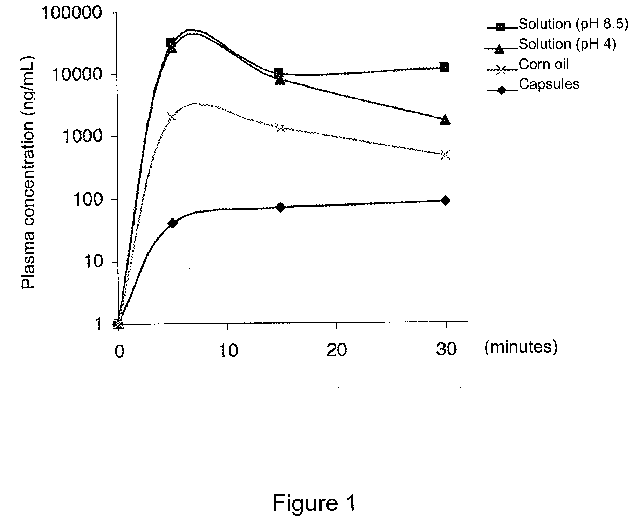

The characteristics and advantages of the present invention will be clear from the detailed description given below, the examples given for illustrative and non-limiting purposes, and the appended Fig. 1, which shows the outcome of the pharmacokinetics tests as set out in Example 7.

### DETAILED DESCRIPTION OF THE INVENTION

The subject of the invention is thus a solution for use as a medicament in oral administration having a pH 7.0 to 9.5, and comprising alpha lipoic acid or its enantiomer, an inorganic compound selected from hydroxide, carbonate, bicarbonate and mixtures thereof, of sodium, potassium, calcium, magnesium, ammonium and mixtures thereof, and a solvent, said solvent being a mixture of water and a polyol selected from propylene glycol, glycerol, polyethylene glycol, polypropylene glycol, and mixtures thereof.

As will also be clear from the Examples provided, this solution has unexpectedly proved to be capable of maintaining the alpha lipoic acid stably dissolved, making it at the same time significantly bioavailable. In addition, said solution has also proved to be advantageously pleasant from an organoeleptic point of view, since, unlike the known alpha lipoic acid formulation, the solution of the invention does not develop any unpleasant smell or flavour, nor precipitates are formed, even after weeks from the preparation. Therefore also the edibility results to be surprisingly and advantageously improved.

In an embodiment of the invention, the solution has a pH 7.0 to 9.5, and consists of alpha lipoic acid or its enantiomer, an inorganic compound selected from hydroxide, carbonate, bicarbonate and mixtures thereof, of sodium, potassium, calcium, magnesium, ammonium and mixtures thereof, and a solvent, said solvent being a mixture of water and a polyol selected from propylene glycol, glycerol, polyethylene glycol, polypropylene glycol, and mixtures thereof.

According to a preferred embodiment, the solution of the invention comprises the *R*-enantiomer of alpha lipoic acid.

Preferably, in said solvent, the polyol is in an amount of 10 to 50% by volume on the volume of the solvent.

More preferably, in said solvent, the polyol is in an amount of 20 to 40% by volume on the volume of the solvent.

According to a preferred embodiment, in said solvent the polyol is in an amount of 25 to 35% by volume on the volume of the solvent. In fact, excellent results are achieved by using these amounts, in terms of stability and bioavailability of the alpha lipoic acid in solution, while at the same time limiting the presence of polyol in favour of water in the solvent, and thus further improving the tolerability and biocompatibility of the final solution.

Preferably, said polyol is propylene glycol, glycerol or a mixture thereof.

More preferably, said polyol is propylene glycol. As will in fact be clear from the Examples given below, propylene glycol has proved to be particularly suitable for the purposes of the present invention, especially in terms of stability of alpha lipoic acid over time.

Preferably, the pH is 8.0 to 9.0, more preferably it is about 8.5.

Preferably, alpha lipoic acid or its enantiomer is present in the solution of the invention at a concentration of 5 to 130 mg/ml, more preferably of 30 to 120 mg/ml, even more preferably of 50 to 80 mg/ml.

According to a preferred embodiment, said concentration is of about 60 mg/ml.

In the solution of the invention, said inorganic compound is preferably selected from hydroxide, carbonate, bicarbonate and mixtures thereof, of sodium, potassium, ammonium and mixtures thereof; more preferably said inorganic compound is hydroxide, carbonate or bicarbonate, of sodium or potassium.

According to a preferred embodiment, when the cation of the inorganic compound is monovalent, i.e. when it is selected from sodium, potassium and ammonium, said inorganic compound is in an equimolar amount to said alpha lipoic acid or its enantiomer.

According to a preferred embodiment, in the solution of the invention, the polyol is in an amount of 25 to 35% by volume on the volume of the solvent, the alpha lipoic acid or its enantiomer is in a concentration of 50 to 80 mg/ml, and the pH is 8.0 to 9.0. In fact, said embodiment allows to achieve an unexpectedly high stability of alpha lipoic acid in the solution of the invention at room temperature over several months, therefore advantageously, without the need to refrigerate during storage.

Within this embodiment, a solution is further preferred wherein the polyol is propylene glycol in an amount of about 30% by volume on the volume of the solvent, alpha lipoic acid or its enantiomer is in a concentration of about 60 mg/ml, and the pH is about 8.5.

Optionally, the solution of the invention can further comprise a basic pH adjuster, which can be selected from the above-reported inorganic compounds.

Optionally, with the aim of further improving the palatability of the solution of the invention, a sweetener, such as aspartame, acesulfame K, sodium saccharine, stevioside or mixtures thereof, may be added, or a flavouring agent, such as orange, anise, black cherry, aloe, banana, caramel, coconut, cherry, alpine herb mix, strawberry, wild berries, tropical fruits, lemon, liquorice, mint, mango, peach, rhubarb, vanilla, or mixtures thereof.

In another aspect, the solution of the invention is in the form of a unit dose comprising 300 to 1200 mg of alpha lipoic acid or its enantiomer.

Preferably, said unit dose comprises 500 to 800 mg of alpha lipoic acid or its enantiomer.

According to a preferred embodiment, said unit dose comprises about 600 mg of alpha lipoic acid or its enantiomer.

According to another preferred embodiment, the solution of the invention is in the form of a unit dose comprising the enantiomer *R* of alpha lipoic acid, which advantageously allows to reduce the doses to be administered. Thus, in said preferred embodiment, the solution of the invention is in the form of a unit dose comprising 150 to 600 mg of alpha (*R*)-lipoic acid. More preferably, said unit dose comprises 150 to 300 mg of alpha (*R*)-lipoic acid.

In a further aspect, the present disclosure concerns a pharmaceutical composition comprising the above-described solution and pharmaceutically acceptable excipients.

The term "excipient" relates to a vehicle, carrier, diluent or adjuvant with which the solution of the invention is administered. Each excipient suitable for the form of preparation desired for the administration is contemplated for use with the solution of the above-described invention.

The excipient can take on a variety of forms depending on the form of preparation desired for the administration. In the preparation of the compositions for the oral dosage form, any of the usual pharmaceutical or food excipients can be used, such as for example, water, glycols, oils, alcohols, flavouring agents, preservatives, colouring agents, and similar agents in the case of oral, liquid compositions; or vehicles such as starches, sugars, microcrystalline cellulose, diluents, granulating agents, lubricants, binders, disaggregating agents, and similar agents in the case of oral, solid compositions, such as for example, hard or soft capsules.

In certain embodiments, the solution for use of the present invention can be combined as active ingredient in an intimate mixture with a suitable, pharmacologically acceptable excipient according to known pharmaceutical preparation techniques.

According to a preferred embodiment, said pharmaceutical composition further comprises acetyl-L-carnitine, selenium, coenzyme Q, vitamins or mixtures thereof. Advantageously, the selenium is preferably in the form of selenomethionine, more preferably of L-selenomethionine.

In a yet further aspect, the present invention relates to a kit comprising:
i) at least one container comprising the solution for use as a medicament in oral administration as above described;
ii) at least one container comprising pharmaceutically acceptable excipients, acetyl-L-carnitine, selenium, coenzyme Q, vitamins or a mixture thereof; and
iii) an illustrative leaflet,
for the separate, sequential or simultaneous oral administration.

In fact, with the aim to preserve the stability of alpha lipoic acid in solution according to the present invention, it is convenient to keep the solution apart from the other components of the pharmaceutical composition.

According to a preferred embodiment, said kit is in the form of a bottle having a container cap comprising pharmaceutically acceptable excipients, acetyl-L-carnitine, selenium, coenzyme Q, vitamins or a mixture thereof, whereas the bottle comprises the solution of the invention. The bottle containing the solution is thus closed by the container cap, hence preserving the components from both external contamination and reciprocal interaction, until the time of use.

In a further aspect, the present invention concerns the medical use of the solution, in particular in the treatment of diabetes, neuropathies, diabetic nephropathy, diabetic retinopathy, diabetic foot, cataracts, obesity, stroke, traumas, reperfusion injuries, cardiomyopathy, mitochondrial decay, canicolar syndromes, radiculopathies, aging, vascular malfunctions in the elderly, cancer, osteoporosis, Alzheimer's disease, Parkinson's disease, hepatic fibrosis or muscular fatigue.

Without wishing to be bound by any theory, it has in fact been hypothesised that, in the acid environment of the stomach, the basic solution of the invention sets free the alpha lipoic acid which, due to the presence of the polyol, remains, at least in part, in solution and is rapidly absorbed. In this way, the time the alpha lipoic acid lasts in the stomach as well as its degradation rate, due to the strongly acidic environment, are thus significantly reduced.

In pharmacokinetics studies conducted by the Applicant and reported in the Examples herein below, comparison tests are performed by administering the alpha-*R*-lipoic acid to rats in solid form or in suspension with corn oil at the dose of 50 mg/kg. In these cases, however, the bioavailability has shown to be disadvantageously reduced, while it was surprisingly observed that the alpha-*R-*lipoic acid dissolved in water/propylene glycol solution according to the present invention (see Figure 1) generates significantly higher peaks of blood concentrations, thus confirming that the bioavailability achieved is decidedly and surprisingly superior.

A series of studies conducted on rats has permitted to show that the cause of the low bioavailability of alpha lipoic acid is essentially a low absorption, due to the following factors: low solubility of alpha lipoic acid in water, on the one hand, and its reduced stability in an acidic environment, such as in the stomach, on the other hand. In support of this hypothesis, the experiments conducted *in vitro* and subsequently described, have demonstrated that alpha lipoic acid has low stability in an acidic solution and that its instability increases by decreasing the pH. Conversely, in a basic solution (7.0<pH<9.5), the alpha lipoic acid is markedly stable. At these pH values, the acid is also more soluble, since it tends, even if slowly at room temperature, to salify. Nevertheless, as mentioned, the presence of a polyol has proven to be essential for improving solubilisation of alpha lipoic acid and thus its delivery, as well as stabilisation in the stomach, where the dramatic decrease of pH would otherwise rapidly deteriorate alpha lipoic acid.

The pharmaceutical solution of the invention for the use as described above is administered by oral route. Advantageously, they can be in the form of vials, bottles, small bottles, syrups, drops, suspensions, capsules or sachets for oral administration.

As will also be clear from the Examples below, said treatment preferably comprises the oral administration of the solution once per day, in particular, of the solution in the form of a unit dose, as described above.

It should be understood that all the aspects identified as preferred and advantageous for the basic solution of the invention are also to be considered similarly preferred and advantageous for its uses, for the pharmaceutical composition and for the kit of the present invention.

Working examples of the present invention are hereinafter provided for illustrative and non-limiting purposes.

### EXAMPLES

### Comparative example 1.

Gelatine capsules, each containing 15 mg of alpha-*R*-lipoic acid, were prepared.

### Comparative example 2.

An oily suspension of alpha-*R*-lipoic acid was prepared in corn oil at a concentration of 5 mg/ml, by shortly sonicating so as to obtain a more uniform suspension.

### Comparative example 3.

An aqueous solution at 70% of propylene glycol was prepared wherein alpha-R-lipoic acid was at a concentration of 5 mg/ml. The pH of this solution was 4.0.

It was observed that, when lower amounts of propylene glycol were used, cloudy solutions were obtained. Furthermore, it was observed that at this pH, irrespective of the amount of propylene glycol, an unpleasant smell and a pungent flavour were caused.

### Example 4.

An aqueous solution at 30% of propylene glycol was prepared wherein alpha-*R-*lipoic acid was present at a concentration of 5 mg/ml. The pH of this solution was brought to 8.5 by adding NaOH 1N.

The resulting solution was subjected to a quantitative stability study and its titre in alpha-R-lipoic acid was monitored for one month. It therefore emerged that the titre advantageously remained essentially constant for the entire period of observation. In addition, during the course of the control month, no cloudiness was observed in the solution, as well as no formation of precipitates or emission of unpleasant smells. For illustrative purposes, Table 1 below sets out the titre and pH values of two samples of the solution obtained according to the present Example. From such values, it was possible to ascertain the stability of the solution of the invention over time.

**Table 1.**

| | **Analysis** | **T=0** | **24 hours** | **10 days** | **20 days** | **30 days** |
|---|---|---|---|---|---|---|
| **Sample A** (5 mg/ml) | **Titre** | 108% | 107% | 108% | 107% | 101% |
| | **pH** | 8.32 | 8.17 | 8.22 | 8.24 | 8.18 |
| **Sample B** (5 mg/ml) | **Titre** | 107% | 107% | 107% | 105% | 100% |
| | **pH** | 8.58 | 8.40 | 8.43 | 8.33 | 8.23 |

The same positive results are also observed 6 months after preparation of the solution.

### Example 5.

Solutions of 600 mg of alpha-*R*-lipoic acid were prepared in variable volumes of propylene glycol from 1 to 5 ml. The solutions were brought to the volume of 10 ml by diluting them with water containing NaOH in an amount stoichiometrically equal to that of the alpha-*R*-lipoic acid present in the solution of propylene glycol. The final solutions, having a concentration of alpha-*R*-lipoic acid of 60 mg/ml and a pH of about 7.5, were limpid, stable and free of any unpleasant smell and pungent flavour.

### Example 6.

The solution prepared according to Example 5, containing 600 mg of alpha-*R-*lipoic acid in 3 ml of propylene glycol with the addition of 7 ml of water (concentration of alpha-*R*-lipoic acid of 60 mg/ml), containing a stoichiometric amount (in relation to the alpha-*R*-lipoic acid) amount of NaOH, was limpid, stable and free of any unpleasant smell and pungent flavour. The pH was adjusted to a value of 8.5 with NaOH 1N. The resulting solution was subjected to a quantitative stability study and its titre in alpha-*R*-lipoic acid was monitored for two months. It therefore emerged that, advantageously, the titre remained essentially constant for the entire period of observation. In addition, during the course of the two control months, no cloudiness was observed in the solution, as well as no formation of precipitates or emission of unpleasant smells. For illustrative purposes, Table 2 below sets out the titre and pH values of two samples of the solution obtained according to the present Example. From such values, it was possible to ascertain the stability of the solution of the invention over time.

**Table 2.**

| | **Analysis** | **T=0** | **24 hours** | **10 days** | **20 days** | **30 days** | **60 days** |
|---|---|---|---|---|---|---|---|
| **Sample A** (**60** mg/ml) | **Titre** | 107% | 106% | 101% | 105% | 102% | **100%** |
| | **pH** | 8.36 | 8.21 | 8.31 | 8.28 | 8.21 | **8.15** |
| **Sample B** (**60** mg/ml) | **Titre** | 106% | 104% | 103% | 103% | 102% | **102%** |
| | **pH** | 8.61 | 8.43 | 8.44 | 8.36 | 8.28 | **8.07** |

The same positive results are also observed 6 months after preparation of the solution.

The stability of this basic solution was also qualitatively assessed by varying the values of its pH from 7.0 to 9.5 by subsequent additions of NaOH 1N or HCl 1N. The solutions thus modified were limpid, free of precipitate and free of any unpleasant smell or pungent flavour.

It was observed that, when the basic solution was instead brought to a pH inferior to 5.0, it immediately developed an unpleasant smell, became cloudy and tended to produce precipitates.

### Example 7.

### Pharmacokinetics studies in rats

In order to perform the pharmacokinetics studies in the rat (with reference to Figure 1) the following formulations were selected:
a) "capsules": capsules as prepared in comparative Example 1;
b) "corn oil": oily suspension of alpha-*R*-lipoic acid in corn oil as prepared in comparative Example 2;
c) "solution (pH 4)": aqueous solution of alpha-*R*-lipoic acid at 70% of propylene glycol having pH 4.0 as prepared in comparative Example 3;
d) "solution (pH 8.5)": aqueous solution of alpha-*R*-lipoic acid at 30% of propylene glycol having pH 8.5 as prepared in Example 4 according to the present invention.

The above formulations were administered by gavage at a dose of alpha-*R*-lipoic acid equal to 50 mg/kg/rat. Groups of 3 rats were used for each formulation analysed. The blood was collected from the caudal vein at 0, 5, 15 and 30 minutes from the administration; it was collected in polypropylene tubes containing heparin as anticoagulant, centrifuged at room temperature, and the plasma sample recovered were analysed for the determination of alpha-*R*-lipoic acid by means of the LC-MS/MS method.

Table 3 below reports the plasma concentrations (ng/ml), following oral administration to the rats, by comparing the results of the capsules of comparative Example 1 with the results of the solution of the invention of Example 4.

**Table 3.**

| minutes | 0 | 5 | 15 | 30 |
|---|---|---|---|---|
| Capsules (Comp. Ex. 1) | 0 (ng/ml) | 41 (ng/ml) | 72 (ng/ml) | 90 (ng/ml) |
| Solution pH 8.5 (Ex. 4) | 0 (ng/ml) | 31352 (ng/ml) | 9976 (ng/ml) | 11712 (ng/ml) |

Table 4, on the other hand, reports the maximum concentration value (Cₘₐₓ) and the time at which it was observed (Timeₘₐₓ) for the capsules of comparative Example 1 and for the solution of Example 4.

**Table 4.**

| Timeₘₐₓ (min) | 5 | 30 |
|---|---|---|
| Capsules (Comp. Ex. 1) | | Cₘₐₓ = 90 (ng/ml) |
| Solution pH 8.5 (Ex. 4) | Cₘₐₓ = 31352 (ng/ml) | |

It was observed, that for the solution of Example 4, the maximum concentration value was obtained 5 minutes after administration (Cₘₐₓ = 31352 ng/ml). On the other hand, for the capsules used as comparison, the maximum concentration value was observed 30 minutes after administration and presented decidedly lower values (Cₘₐₓ = 90 ng/ml).

As could be seen from Table 4, the Cₘₐₓ of the solution of Example 4 of the invention was surprisingly of two orders of magnitude (order of magnitude 10⁴) higher than the one observed for the capsules of comparative Example 1 (order of magnitude 10²).

With particular reference to Fig. 1, the pharmacokinetics profile was reported, as obtained with an oral administration in rats (dose: 50 mg/Kg/rat) of alpha-R-lipoic acid prepared according to the different, previously-described formulations a) to d). It was observed, in addition to what already discussed for Tables 3 and 4, that the formulation c) "solution (pH 4)" also presented a high plasma concentration (Cₘₐₓ = 27600 ng/ml), however this formulation was not definitely limpid, thus indicating that the solubility of alpha-*R*-lipoic acid therein was not complete. In fact, the pharmacokinetics profile in Figure 1 rapidly decreases in the subsequent 25 minutes (from 27600 ng/ml to 1767 ng/ml). In addition, an unpleasant smell developed that also made the "solution (pH 4)" disadvantageous under the organoleptic point of view.

With the formulation b) however, the results were even worse than those of formulation c), since in the corn oil suspension, alpha-*R*-lipoic acid presented a much lower bioavailability, with a Cₘₐₓ = 2070 ng/ml after 5 minutes.

### Example 8.

Five samples of the solution of the invention were prepared according to the procedure of Example 5, at different propylene glycol concentrations, but at a concentration of alpha-R-lipoic acid of 60 mg/ml and a sample at a concentration of alpha-R-lipoic acid of 120 mg/ml, as follows:

| Sample | R-αLA (mg/ml) | % Propylene glycol | pH |
|---|---|---|---|
| 1 | 60 | 10% | 8.5 |
| 2 | 60 | 20% | 8.5 |
| 3 | 60 | 30% | 8.5 |
| 4 | 60 | 40% | 8.5 |
| 5 | 60 | 50% | 8.5 |
| 6 | 120 | 30% | 8.5 |

Table 5 below reports the results achieved from a further quantitative stability study, by monitoring, for various time intervals, the titre of alpha-R-lipoic acid in the above samples.

**Table 5.**

| Sample | Analysis | T=0 | 7 days | 40 days | 70 days | 90 days | 6 months |
|---|---|---|---|---|---|---|---|
| 1 | Titre | 52% | 53.1% | 55.9% | 57.9% | 63.1% | 63.3% |
| | pH | 8.51 | 8.45 | 8.05 | 8.08 | 7.88 | 7.59 |
| 2 | Titre | 71% | 63.4% | 63.0% | 64.3% | 65.6% | 67.5% |
| | pH | 8.48 | 8.52 | 8.16 | 8.13 | 7.71 | 7.63 |
| 3 | Titre | 106% | 102.8% | 97.3% | | 93.6% | 86.3% |
| | pH | 8.56 | 8.66 | 8.18 | | 7.85 | 7.63 |
| 4 | Titre | 113% | 103.3% | 102.9% | | 98.4% | 93.5% |
| | pH | 8.59 | 8.54 | 8.06 | | 7.96 | 7.71 |
| 5 | Titre | 119% | 105.7% | 108.5% | 102.4% | 100.8% | 101.9% |
| | pH | 8.54 | 8.50 | 8.22 | 8.18 | 8.04 | 7.82 |
| 6 | Titre | 83% | 63.0% | 48.6% | | 49.7% | 45.8% |
| | pH | 8.55 | 8.60 | 8.33 | | 8.03 | not det. |

Also in these tests, no traces of degradation of the active ingredient or of the formation of precipitates or unpleasant smells were observed, that are usually ascribable to the reduction of lipoic acid into dihydrolipoic acid. On analysing the trend of the titre of the lipoic acid, it was observed that it remained substantially constant with respect to the initial value, thus confirming that in the solvent used the stability of the lipoic acid itself was preserved.

A particular aspect that was highlighted by these results was the fact that the use of concentrations of propylene glycol lower than 30% determined a reduced titre of lipoic acid already from the starting point of the analysis (i.e. T=0), a titre that however remained essentially constant throughout the period considered. On the contrary, at higher concentrations of propylene glycol, for example at 50%, higher titres of lipoic acid than expected were obtained. The cause of these observed phenomena has yet to be identified; from the several tests conducted (HPLC, differential scanning calorimetry, etc.), it has however been hypothesised that the propylene glycol could determine some intermolecular interactions with the lipoic acid, interactions that, albeit weak, could interfere with analysis of the titre of the active ingredient.

Lastly, in considering the stability of a formulation obtained by using a higher dose of R-αLA acid (120 mg/ml of sample 6), it was highlighted that even in this case the titre appeared reduced already from the initial analysis, but the most evident decrease was observed over the first 40 days. This phenomenon could at the same time be explained on the basis of the intermolecular interactions that could take place between lipoic acid and propylene glycol and thus from the ratio thereof present within the formulation.

From what reported above, the preferred solution was that of sample 3, wherein the R-αLA acid was in a concentration of 60 mg/ml in aqueous solution at 30% of propylene glycol, pH=8.5. With respect to said solution, the stability of lipoic acid was also analysed, by evaluating the titre even after longer time intervals (up to 9 months); for this further analysis, the solution was kept at different temperatures (5°C, 25°C, 30°C e 40°C) and humidity conditions (60% UR, 65% UR e 75% UR), but stored in amber, plastic bottles, to prevent any light damage, as set out in Table 6 below.

**Table 6.**

| Temperature - Humidity | T=0 | T=1 month | T=3 months | T=6 months | T=9 months |
|---|---|---|---|---|---|
| 5°C | 100.80% pH=8.61 | 62.27% pH=8.78 | 57.76% pH=9.00 | 56.69% pH=8.51 | 51.66% pH=8.85 |
| 25°C - 60%U | | 95.61% pH=8.13 | 95.59% pH=7.97 | 93.28% pH=7.48 | 90.27% pH=7.72 |
| 30°C - 65%U | | 96.06% pH=8.03 | 99.80% pH=7.68 | 95.75% pH=7.30 | 96.20% pH=7.65 |
| 40°C - 75%U | | 96.87% pH=7.82 | 100.31% pH=7.37 | 99.43% pH=7.01 | 98.52% pH=7.34 |

As reported in Table 6 above, a dramatic decrease in the titre was only observed in the solutions stored at +5°C, a reduction that was very evident, especially after the first month, when a titre of 62.72% was achieved compared to the titre of 100.80% recorded at the starting time.

In all the other temperature conditions tested (associated with different humidity values), even 9 months after the preparation, significantly high values of the titre of the lipoic acid were maintained, close to the initial valued, thus confirming the stability of the active ingredient in this solution. These physicochemical observations allowed to ascertain that the greater stability of the lipoic acid in the solution of the invention was unexpectedly at room temperature, so as advantageously without refrigerating.

### Example 9.

### Solubility of the R-alpha lipoic acid of the invention in gastric juice

Example 7 evaluated the increase in the bioavailability of lipoic acid in the solution of the invention compared to the lipoic acid administered in solid form. It was hypothesised that this result, which allowed certain limits in the use of lipoic acid (low bioavailability and short half-life) to be overcome, was achieved in that with the solution thus formulated the solubility of the lipoic acid was improved so that to create, as a consequence, optimal conditions for increasing absorption at gastric level.

It was possible to demonstrate an improved solubility of lipoic acid formulated in aqueous solution containing propylene glycol through analysis capable of reproducing in vitro conditions that occur during gastric digestion in humans. In this perspective, lipoic acid, both in racemic form and in enantiomeric form *R*, used as such, therefore in powder (solid form), or formulated in aqueous solution containing different concentrations of propylene glycol, was dissolved in a volume of 500 ml of artificial gastric juice, prepared according to the European Pharmacopoeia 7.0, and kept under stirring so as to mimic human gastric digestion as far as possible. The amounts of lipoic acid administered (600 to 300 mg) refer to daily doses used in various clinical studies.

From analysis of the physical parameters observed, it could be deduced that only with the solution of the invention was there obtained a homogenous mixture, as shown in Table 7 below.

**Table 7.**

| | α-LA acid | | Propylene glycol | Observations |
|---|---|---|---|---|
| | form | amount | | |
| | | (mg) | (%) | |
| a | RS | 600 | 0 | flocculent precipitate with clear supernatant |
| b | R | 600 | 0 | presence of compact aggregate in a slightly cloudy liquid |
| | | | | |
| c | RS | 600 | 30 | slightly opalescent suspension; very rare undissolved particles |
| d | R | 600 | 30 | cloudy suspension without precipitate |
| e | R | 600 | 10 | slightly opalescent suspension with yellow precipitate |
| f | R | 300 | 30 | slightly opalescent solution without precipitate |

In all the other situations, especially in the presence of lipoic acid in solid form, precipitates of different extent were observed adhering to the bottom of the flask or to the stirrer.

The above-described visual indications highlighted the different solubilities of lipoic acid in gastric juice depending on whether used in the form of powder or of a liquid solution.

In order to confirm what reported above and to verify the advantages of the solution of the invention, a comparative study was also conducted with different food supplements containing lipoic acid, in racemic or enantiomeric *R* form, available on the market in tablets or coated granules (samples A-E). In this case also, the daily dose of lipoic acid was dissolved in artificial gastric juice prepared according to the European Pharmacopoeia 7.0, and kept under stirring for a number of hours to mimic human gastric digestion. The results of this study are reported in Table 8.

**Table 8.**

| Sample | α-LA acid | | Propylene glycol | Observations |
|---|---|---|---|---|
| | form | amount | | |
| | | (mg) | (%) | |
| A | RS | 600 | 0 | yellow suspension, due to the dyes, containing bottom sediment |
| B | RS | 600 | 0 | clear suspension, with the presence of significant undissolved corpuscles at the bottom |
| C | R | 600 | 0 | essentially undissolved tablet, in a clear solution |
| D | RS | 600 | 0 | significant presence of granules at the bottom in a clear supernatant |
| E | RS | 600 | 0 | significant presence of granules on the bottom in a viscous suspension for the excipients used |
| d (of Table 7) | R | 600 | 30 | homogeneous, but opalescent, suspension |
| f (of Table 7) | R | 300 | 30 | limpid, slightly opalescent, suspension |

As reported in Table 8, in the case of the different food supplements available on the market (A-E), as a result of the *in vitro* digestion, a considerable sediment was observed at the bottom of the flask, a phenomenon which, could *inter alia* be the cause of the gastric disorders. Part of lipoic acid was surely dissolved in the gastric juice, in that it was extracted from the phase recovered following necessary centrifugation to separate the sediment (data not shown); however, most of lipoic acid remained in insoluble form. On the contrary, in the case of digestion in gastric juice of the solutions of the invention (samples 'd' and 'f'), a homogeneous mixture was obtained, with a degree of opalescence dependant on the amount of dissolved alpha lipoic acid, but without precipitate.

In essence, the increase in the bioavailability recorded for lipoic acid dissolved in the solution of the invention was in line with the solubility of lipoic acid in a gastric environment, the region in which its maximum absorption occurs.

### Example 10.

### Biological activity on cell cultures of alpha lipoic acid in the solution of the invention

Lipoic acid has several biological functions; among the most documented, it should be reminded that it is a known cofactor of enzymatic decarboxylation reactions, an antioxidant capable of regenerating endogenous defence systems, a regulator of both the transcription of different Nfr2-dependant genes and the glucose metabolism. It follows that the improvement of the bioavailability of lipoic acid inherently influences all those biological functions wherein it plays an active role. Through a series of experiments on cell cultures, it was studied whether the solution of the invention modifies the biological activity of lipoic acid; for this reason, some of its target parameters, such as the cell vitality index, the ability to reduce the amount of ROS (reactive oxygen species) induced from H₂O₂ and that of promoting glucose uptake were monitored.

For the following tests, an alpha lipoic acid solution at a concentration of 60 mg/ml, in propylene glycol at 30% and at pH 8.5 was used.

A lipoic acid concentration, previously identified as effective in reducing the biological activity under examination, was added to the culture medium of the cell lines used.

In fact, from previous cell vitality tests, it was repeatedly demonstrated that at the 1mM concentration, a 48-hr *R*-lipoic acid treatment, dissolved in DMSO, determined a significant cessation of the proliferation of the HepG2 cells. Under these conditions, the cell vitality values, obtained by means of the ATP lite kit (PerkingElmer), resulted to be halved compared to the control, which was only treated with the solvent used for the lipoic acid (DMSO).

In order to analyse the effects of the solution of the invention, alpha lipoic acid contained therein was digested in artificial gastric juice and subsequently administered in the culture medium at the final concentration of 0.5 or 1mM. The cell vitality effects recorded were compared with those achieved in a parallel control experiment wherein alpha lipoic acid dissolved in a solvent of election for cell studies, i.e. DMSO (but not digested in gastric juice) was used. As highlighted by the values recorded in Table 9, achieved 3×24 hr after the start of treatment with the various solutions being analysed, at the concentration of 0.5mM of lipoic acid from the solution of the invention, cell vitality values comparable to those recorded for lipoic acid 1mM dissolved in DMSO, were observed. The different effects observed at the same dose administered indicated a greater intracellular amount of lipoic acid, if formulated in accordance with the present invention, thus strengthening the fact that at the improved solubility in gastric juice as described above, an increase in cellular absorption was advantageously associated.

**Table 9.**

| | [conc.]_{final} (mM) | Vitality %) |
|---|---|---|
| Ctrl-1 | | 100 |
| (RS) alphaLA (in DMSO) | 1.0 | 35.48 |
| (R) alphaLA (in DMSO) | 1.0 | 40.33 |
| Ctrl-2 | | 100 |
| (RS) alphaLA (of the invention) | 0.5 | 52.59 |
| | 1.0 | 14.70 |
| (R) alphaLA (of the invention) | 0.5 | 46.90 |
| | 1.0 | 16.99 |

| | | |
|---|---|---|
| where Ctrl-1: DMSO 1%; Ctrl-2: aqueous solution at 30% of propylene glycol. | | |

The achieved results referred to 72-hour (3x24 hr) treatments on HepG2 cells, but were also confirmed in other cell lines of human origin, such as the neuroblastoma SK-N-SH cell lines.

From these indications obtained from cell studies, it was highlighted that, by the solution of the invention, it was possible to advantageously decrease the daily dose of lipoic acid, while maintaining a significant biological effect.

Another important function ascribable to lipoic acid is that of contrasting the formation of reactive species of oxygen induced from H₂O₂. Through tests on SK-N-SH cell cultures, it was repeatedly demonstrated that the quantity of H₂O₂-dependent ROS, produced after a treatment with alpha lipoic acid, decreased, thus indicating that different mechanisms were activated in order to detoxify and/or prevent the formation of these reactive species that are detrimental for cell survival. A further aim was to verify whether *R*-alpha lipoic acid retained this function also in an aqueous solution. For this reason, increasing doses of the same, coming from the above-used solution of the invention and subjected to digestion in artificial gastric juice, were added to the culture medium for different intervals of time (24 hr and 48 hr) before inducing an oxidative stress with H₂O₂. The same reaction was performed as a control, by using the *R*-alphaLA prepared in DMSO, but dissolved directly in medium to obtain the same working concentrations.

**Table 10.**

| | Propylene glycol (%) | Ros (% compared to ctrl) | |
|---|---|---|---|
| | | 24h | 48h |
| Ctrl-1 | 0 | 100.00 | 122.21 |
| R-alphaLA 100µM (DMSO) | 0 | 77.87 | 99.26 |
| R-alphaLA 200µM (DMSO) | 0 | 49.18 | 103.98 |
| | | | |
| Ctrl-2 | 30 | 100.00 | 113.76 |
| R-alphaLA 100µM (solut. of the invention) | 30 | 75.56 | 54.43 |
| R-alphaLA 200µM (solut. of the invention) | 30 | 66.77 | 58.51 |

| | | | |
|---|---|---|---|
| Ctrl-1: DMSO 1%; Ctrl-2: aqueous solution at 30% of propylene glycol. | | | |

In Table 10, the values of ROS are reported as % with respect to those observed in the controls after 24 hours of maintenance in the culture and 2 hours of treatment with 1.5mM mM H₂O₂. In the first box of the Table, the results are shown as obtained after administration of R-alphaLA prepared in DMSO and then diluted directly in the medium to obtain the reference final concentrations; in the second box, on the other hand, R-alphaLA was first dissolved in the solution of the invention, subsequently digested in gastric juice and finally diluted in the medium. As summarised in Table 10, in each case, a dose-dependent response of alpha lipoic acid in preventing the formation of the ROS was observed over the first 24 hours. At later times (48 hours), it was noted, as expected, an increased basal value of ROS in the control, due to the physiological conditions of maintenance in the culture, but, surprisingly, only the lipoic acid coming from the solution of the invention continued to act an ROS-detoxification function.

Lastly, the hypoglycaemia-inducing action of the lipoic acid formulated in aqueous solution of the invention was also evaluated, by utilising an insulin-resistant diabetes cellular model. HepG2 cells cultivated in a medium enriched with glucose and free of insulin mimicked certain conditions present in the hepatocytes of diabetic patients; in addition, certain functions of the culture medium, considered as a container of nutritional substances and an accumulation container for those substances excreted, could be compared to plasma circulation. This cellular model allowed to demonstrate that lipoic acid promoted a significant decrease of glucose from the medium, consequent to a greater expression of the membrane transporters of glucose as well as an inhibition of gluconeogenesis.

**Table 11.**

| | Propylene glycol (%) | Glucose in the medium (%) |
|---|---|---|
| Ctrl-1 | 0 | 100 |
| (*R*) alphaLA 1 mM (DMSO) | 0 | 89 |
| | | |
| Ctrl-2 | 30 | 100 |
| (*R*) alphaLA 1 mM (solut. of the invention) | 30 | 79 |

| | | |
|---|---|---|
| Ctrl-1: DMSO 1%; Ctrl-2: aqueous solution at 30% of propylene glycol. Arbitrary unit (U = 100%): amount of glucose present in the medium of the reference control. Cellular model of insulin-resistant diabetes: HepG2 cultivated in a medium enriched with glucose 30mM | | |

Table 11 reports some of the most significant results achieved by using the Sigma Aldrich Glucose assay kit to determine the glucose; more specifically, the values obtained from the culture medium following administration for 48 hr of lipoic acid (1mM) prepared in DMSO or formulated in aqueous solution at 30 % of propylene glycol, in accordance with the present invention. The most significant decrease in glucose was in fact observed with the *R*-enantiomer of alpha lipoic acid in the solution of the invention.

The advantages achieved by the basic solution of the present invention are clear from the detailed description and the above-reported Examples. In particular, this composition has surprisingly and advantageously proven to be capable of stabilising and making extremely biodegradable the alpha lipoic acid, owing to the appropriate combination of pH and of solvents. In addition, the solution of the invention has also been surprisingly advantageous under an organoeleptic point of view, since said solution is limpid and does not develop unpleasant smells or flavours, being therefore extremely improved even in terms of edibility.

## Claims

1. Solution for use as a medicament in oral administration, said solution having a pH of 7.0 to 9.5, being an homogenous mixture, and comprising the compound alpha lipoic acid or its enantiomer, an inorganic compound selected from hydroxide, carbonate, bicarbonate and mixtures thereof, of sodium, potassium, calcium, magnesium, ammonium and mixtures thereof, and a solvent, said solvent being a mixture of water and a polyol selected from propylene glycol, glycerol, polyethylene glycol, polypropylene glycol, and mixtures thereof.

2. The solution for use of claim 1, wherein in said solvent the polyol is in an amount of 10 to 50% by volume on the volume of the solvent.

3. The solution for use of claim 2, wherein in said solvent the polyol is in an amount of 20 to 40% by volume on the volume of the solvent, preferably of 25 to 35% by volume on the volume of the solvent.

4. The solution for use of any one of claims 1-3, wherein said polyol is propylene glycol, glycerol or a mixture thereof.

5. The solution for use of claim 4, wherein said polyol is propylene glycol.

6. The solution for use of any one of claims 1-5, wherein said compound alpha lipoic acid or its enantiomer is at a concentration of 5 to 130 mg/ml, preferably of 30 to 120 mg/ml, more preferably of 50 to 80 mg/ml.

7. The solution for use of any one of claims 1-6, wherein said inorganic compound is selected from hydroxide, carbonate, bicarbonate and mixtures thereof, of sodium, potassium, ammonium and mixtures thereof.

8. The solution for use of claim 7, wherein said inorganic compound is in an equimolar amount to said compound alpha lipoic acid or its enantiomer.

9. The solution for use of claim 8, further comprising a basic pH adjuster.

10. The solution for use of any one of claims 1-9, in the form of a unit dose comprising 300 to 1200 mg of alpha lipoic acid or its enantiomer, preferably 500 to 800 mg.

11. The solution for use of any one of claims 1-9, in the form of a unit dose comprising 150 to 600 mg of (*R*) alpha lipoic acid, preferably 150 to 300 mg.

12. The solution for use of any one of claims 1-11 in the treatment of diabetes, neuropathies, diabetic nephropathy, diabetic retinopathy, diabetic foot, cataracts, obesity, stroke, trauma, reperfusion injuries cardiomyopathy, canicolar syndromes, radiculopathies, aging, vascular malfunction in the elderly, cancer, osteoporosis, Alzheimer's disease, Parkinson's disease, hepatic fibrosis or muscular fatigue.

13. Kit comprising:
i) at least one container comprising the solution for use as a medicament in oral administration of any one of claims 1-11;
ii) at least one container comprising pharmaceutically acceptable excipients, acetyl-L-carnitine, selenium, coenzyme Q, vitamins or a mixture thereof; and
iii) an illustrative leaflet,
for the separate, sequential or simultaneous oral administration.

## Patentansprüche

1. Lösung zur Verwendung als Medikament zur oralen Verabreichung, wobei die Lösung einen pH-Wert zwischen 7,0 und 9,5 aufweist, ein homogenes Gemisch ist, die Verbindung Alpha-Liponsäure oder deren Enantiomer, eine anorganische Verbindung ausgewählt aus Hydroxid, Karbonat, Bikarbonat und Mischungen davon, sowie Natrium, Kalium, Kalzium, Magnesium, Ammonium und Mischungen davon, und ein Lösungsmittel aufweist, wobei das Lösungsmittel eine Mischung von Wasser mit einem Polyol ist, das ausgewählt ist aus Propylenglykol, Glyzerin, Polyethylenglykol, Polypropylenglykol und Mischungen davon.

2. Lösung zur Verwendung gemäß Anspruch 1, wobei das Polyol in dem Lösungsmittel in einer Menge von 10 bis 50 Vol.-% des Volumens des Lösungsmittels vorliegt.

3. Lösung zur Verwendung gemäß Anspruch 2, wobei das Polyol in dem Lösungsmittel in einer Menge von 20 bis 40 Vol.-% des Volumens des Lösungsmittels vorliegt, vorzugsweise in einer Menge von 25 bis 35 Vol.-% des Volumens des Lösungsmittels.

4. Lösung zur Verwendung gemäß einem der Ansprüche 1 bis 3, wobei das Polyol Propylenglykol, Glyzerin oder eine Mischung davon ist.

5. Lösung zur Verwendung gemäß Anspruch 4, wobei das Polyol Propylenglykol ist.

6. Lösung zur Verwendung gemäß einem der Ansprüche 1 bis 5, wobei die Verbindung Alpha-Liponsäure oder deren Enantiomer in einer Konzentration von 5 bis 130 mg/ml vorliegt, vorzugsweise 30 bis 120 mg/ml, weiter bevorzugt 50 bis 80 mg/ml.

7. Lösung zur Verwendung gemäß einem der Ansprüche 1 bis 6, wobei die anorganische Verbindung ausgewählt wird aus Hydroxid, Karbonat, Bikarbonat und Mischungen davon, sowie Natrium, Kalium, Ammonium und Mischungen davon.

8. Lösung zur Verwendung gemäß Anspruch 7, wobei die anorganische Verbindung in einer äquimolaren Menge zu der Verbindung Alpha-Liponsäure oder deren Enantiomer vorliegt.

9. Lösung zur Verwendung gemäß Anspruch 8, weiter aufweisend einen basischen pH-Wert-Regulator.

10. Lösung zur Verwendung gemäß einem der Ansprüche 1 bis 9, in Form einer Einzeldosis mit 300 bis 1.200 mg Alpha-Liponsäure oder deren Enantiomer, vorzugsweise 500 bis 800 mg.

11. Lösung zur Verwendung gemäß einem der Ansprüche 1 bis 9, in Form einer Einzeldosis mit 150 bis 600 mg (R) Alpha-Liponsäure, vorzugsweise 150 bis 300 mg.

12. Lösung zur Verwendung gemäß einem der Ansprüche 1 bis 11 bei der Behandlung von Diabetes, Neuropathien, diabetischer Nephropathie, diabetischer Retinopathie, diabetischem Fuß, Katarakten, Adipositas, Schlaganfall, Trauma, Reperfusionsschäden, Kardiomyopathie, Canicolar-Syndromen, Radikulopathie, Alterung, vaskulärer Störung bei älteren Menschen, Krebs, Osteoporose, Alzheimer, Parkinson, Leberfibrose oder Muskelermüdung.

13. Set mit:
i) zumindest einem Behälter mit der Lösung zur Verwendung als Medikament zur oralen Verabreichung gemäß einem der Ansprüche 1 bis 11;
ii) zumindest einem Behälter mit pharmazeutisch akzeptablen Hilfsstoffen, Acetyl-L-Carnitin, Selen, Koenzym Q, Vitaminen oder einer Mischung davon; und
iii) einem erläuterndem Merkblatt für die einzelne, die sequenzielle oder die simultane orale Verabreichung.

## Revendications

1. Solution pour une utilisation en tant que médicament en administration orale, ladite solution ayant un pH de 7,0 à 9,5, étant un mélange homogène, et comprenant le composé d'acide alpha-lipoïque ou son énantiomère, un composé inorganique choisi parmi l'hydroxyde, le carbonate, le bicarbonate et leurs mélanges, de sodium, de potassium, de calcium, de magnésium, d'ammonium et de leurs mélanges, et un solvant, ledit solvant étant un mélange d'eau et d'un polyol choisi parmi le propylène glycol, le glycérol, le polyéthylène glycol, le polypropylène glycol, et leurs mélanges.

2. Solution pour une utilisation selon la revendication 1, dans laquelle dans ledit solvant le polyol est dans une quantité de 10 à 50 % en volume sur le volume du solvant.

3. Solution pour une utilisation selon la revendication 2, dans laquelle dans ledit solvant le polyol est dans une quantité de 20 à 40 % en volume sur le volume du solvant, de préférence de 25 à 35 % en volume sur le volume du solvant.

4. Solution pour une utilisation selon l'une quelconque des revendications 1 à 3, dans laquelle ledit polyol est le propylène glycol, le glycérol ou l'un de leurs mélanges.

5. Solution pour une utilisation selon la revendication 4, dans laquelle ledit polyol est le propylène glycol.

6. Solution pour une utilisation selon l'une quelconque des revendications 1 à 5, dans laquelle ledit composé d'acide alpha-lipoïque ou son énantiomère est à une concentration de 5 à 130 mg/ml, de préférence de 30 à 120 mg/ml, de façon davantage préférée de 50 à 80 mg/ml.

7. Solution pour une utilisation selon l'une quelconque des revendications 1 à 6, dans laquelle ledit composé inorganique est choisi parmi l'hydroxyde, le carbonate, le bicarbonate et leurs mélanges, de sodium, de potassium, d'ammonium et de leurs mélanges.

8. Solution pour une utilisation selon la revendication 7, dans laquelle ledit composé inorganique est dans une quantité équimolaire par rapport audit composé d'acide alpha-lipoïque ou son énantiomère.

9. Solution pour une utilisation selon la revendication 8, comprenant en outre un agent d'ajustement de pH basique.

10. Solution pour une utilisation selon l'une quelconque des revendications 1 à 9, sous la forme d'une dose unitaire comprenant 300 à 1200 mg d'acide alpha-lipoïque ou de son énantiomère, de préférence 500 à 800 mg.

11. Solution pour une utilisation selon l'une quelconque des revendications 1 à 9, sous la forme d'une dose unitaire comprenant 150 à 600 mg d'acide (*R*)-alpha-lipoïque, de préférence 150 à 300 mg.

12. Solution pour une utilisation selon l'une quelconque des revendications 1 à 11 dans le traitement du diabète, de neuropathies, de la néphropathie diabétique, de la rétinopathie diabétique, du pied diabétique, de cataractes, de l'obésité, de l'accident vasculaire cérébral, d'un traumatisme, de lésions de reperfusion, d'une cardiomyopathie, de syndromes canicolaires, de radiculopathies, du vieillissement, d'un dysfonctionnement vasculaire chez la personne âgée, du cancer, de l'ostéoporose, de la maladie d'Alzheimer, de la maladie de Parkinson, de la fibrose hépatique ou de la fatigue musculaire.

13. Kit comprenant :
i) au moins un récipient comprenant la solution pour une utilisation en tant que médicament en administration orale selon l'une quelconque des revendications 1 à 11 ;
ii) au moins un récipient comprenant des excipients pharmaceutiquement acceptables, de l'acétyl-L-carnitine, du sélénium, du coenzyme Q, des vitamines ou l'un de leurs mélanges ; et
iii) un dépliant explicatif,
pour l'administration orale séparée, séquentielle ou simultanée.
